# EUROPEAN PATENT APPLICATION

(11) **EP 4 776 260 A2**
(43) Date of publication of application: **15.07.2026**
(21) Application number: 26182362.9
(22) Date of filing: 16.04.2021
(51) Int. Cl.: G09B 5/02

(54) **METHOD OF OPERATION OF A COMPUTER ASSISTED REALITY SYSTEM FOR A MEDICAL APPARATUS, COMPUTER ASSISTED REALITY SYSTEM AND COMPUTER PROGRAM PRODUCT**

(62) Divisional of application: 21169009.4
(71) Applicant: Baxter Medical Systems GmbH + Co. KG, 07318 Saalfeld (DE)
(72) Inventor: YAP, Cheah Chang, Batesville, 47006 (US); SCHULZE, Katrin, Batesville, 47006 (US); SCHWADE, Michael, Batesville, 47006 (US)
(74) Representative: Reddie & Grose LLP

(57) **Abstract**

A method of operation for a computer assisted reality system (1) for a medical apparatus is disclosed. The computer assisted reality system (1) comprises the medical apparatus and a smart device (2) having a camera (4) and an illustration device for illustrating an image. The method comprises the steps: determining a type of the medical apparatus by detecting an input of the type to the smart device (2) or by evaluating an image of a real medical apparatus captured by the camera (4); and displaying information (20) concerning the medical apparatus via the illustration device.

## Description

The invention relates to a method of operation of a computer assisted reality system for a medical apparatus, a computer assisted reality system, and a computer program product for executing the method, in particular for E-learning or service utilizations.

Hitherto, learning about and training of an operation of a medical apparatus is done in real surroundings of the medical apparatus by means of a real medical apparatus. However, if such a medical apparatus is already in use, the learning about and training of the operation occupies the medical apparatus so that it is not available for, e.g., surgical interventions. On the other hand, in case that the medical apparatus is not yet available in, e.g., a hospital, access of a training facility is necessary which is not desirable, in particular, during a pandemic crisis.

Moreover, for service or maintenance, in particular, also for service and maintenance training, it is necessary to be present at the medical apparatus and to have access to the documents necessary for the service or maintenance. For determining a state of the medical apparatus or of components thereof, it is necessary to open or dismantle the medical apparatus. In some cases, several service calls are necessary since, in particular in a service case, necessary spare parts are not immediately available.

Therefore, the object underlying the invention is to improve a proper handling with the medical apparatus by improving the learning about and the training of the operation of the medical apparatus and by enhancing a situation in maintenance and service calls.

The object is achieved by a method according to clause 1, a computer assisted reality system according to clause 20, and a computer program product according to clause 22. Advantageous further developments are included in the dependent clauses.

According to an aspect of the invention, a method of operation for a computer assisted reality system for a medical apparatus, wherein the computer assisted reality system comprises the medical apparatus and a smart device having a camera and an illustration device for illustrating an image, comprises the steps: determining a type of the medical apparatus by detecting an input of the type to the smart device or by evaluating an image of a real medical apparatus captured by the camera; and displaying information concerning the medical apparatus via the illustration device.

The computer assisted reality system comprising the smart device enables watching the medical apparatus via the smart device, e.g., smart glasses, a smart phone or a tablet, and displaying information concerning the medical apparatus via the smart device. For performing this task, a computer program product, a so-called application, is executed by the smart device.

For displaying the information concerning the medical apparatus via the illustration device of the smart device, at least a type of the medical apparatus has to be determined. This can be done by the input of the type to the smart device or by the evaluation of the image captured by the camera.

By these steps, the basis for learning about and training of the medical apparatus and for enhanced maintenance or service calls, i.e., the opportunity for input of according instructions, is given so that the proper handling is improved.

In an advantageous implementation of the method, the computer assisted reality system is formed by an augmented reality system and the illustration device is formed by a touchscreen illustrating an image captured by the camera, wherein the input to the smart device is performed via touching the touchscreen by an object, wherein the method comprises the step: illustrating an illustration of the medical apparatus according to the type of the medical apparatus against a backdrop via the illustration device.

By the use of the augmented reality system, the execution of the method can be done with manageable efforts since merely a smart device, such as a tablet computer or a cell phone, is necessary for improving the proper handling of the medical apparatus. **In** the augmented reality system, the image of a real world can be superimposed with additional information on the display of the smart device. Moreover, the smart device captures images by its camera for illustrating the medical apparatus against the backdrop via the illustration device of the smart device and inputs can be performed by touching the touchscreen by the object, e.g., a finger.

According to an advantageous implementation of the method, it further comprises the steps: capturing an image of surroundings of the smart device by the camera, and illustrating an illustration of a 3D model of the type of the medical apparatus in the image of the surroundings being the backdrop via the illustration device.

When the image of the surroundings of the smart devices captured by the camera and illustrated by the illustration device and, also, the illustration of the 3D model of the type of the medical apparatus are displayed, the medical apparatus can be illustrated in learner's surroundings. This can be either a real operating place in an operating theater of a hospital or, e.g., in a room of a training facility or in the home of a person. The 3D model is then displayed in quasi-real operating conditions so that the proper handling of the medical apparatus is improved.

In another advantageous implementation of the method, it further comprises the steps: capturing an image of the medical apparatus by the camera, illustrating the captured image of the real medical apparatus against the backdrop, and determining the type of the medical apparatus by evaluating the captured image of the real medical apparatus.

By this implementation, a manual determination of the type of the medical apparatus is not necessary since, from the captured image of the real medical apparatus, its type can be unambiguously determined. Moreover, the medical apparatus in its actual configuration can be determined and further information resulting thereof can be displayed.

In another advantageous implementation of the method, the computer assisted reality system is formed by a mixed reality system and the illustration device is formed by smart glasses, wherein the input to the smart device is performed via capturing an object and a motion of the object by the camera, wherein the method comprises the step: illustrating an illustration of a 3D model of the type of the medical apparatus via the illustration device.

Due to the use of the mixed reality system comprising the smart glasses which enables watching the real surrounding and illustrating the 3D model of the type of the medical apparatus as a hologram, a more sophisticated impression of the medical apparatus can be achieved and a more exact handling of the medical apparatus is possible since a user virtually touches and operates the medical apparatus almost as in real environments by moving his fingers or hands. In the mixed reality system, the holograms can be placed in a real world so that they seem to be actually present in the observed area. By means of the smart glasses, the real items of an area including the hands of the operator of the system as well as the holograms illustrating desired further items can be observed and the input can be performed by detecting the object, e.g., the hand or fingers.

In a further advantageous implementation of the method, the method further comprises the step: moving the illustration of the 3D model of the type of the medical apparatus by virtually touching the illustration of the 3D model of the type of the medical apparatus by means of an object and by moving the object.

Due to these options, the illustration of the medical apparatus can be moved, e.g., by means of a finger or a hand on the touch screen or captured by the camera, and can be regarded from different sides. Depending on the available degrees of freedom, the medical apparatus can be displaced and/or rotated in order to have the opportunity to regard also views of the medical apparatus which, in real life, would only be possible when the operator himself would move so that the proper handling of the medical apparatus is improved.

According to a further advantageous implementation of the method, the information comprises a virtual keypad of the medical apparatus, and the method further comprises the step: actuating motions of the 3D model of the medical apparatus according to inputs to the virtual keypad.

The virtual keypad of the medical apparatus enables an operation according to the operation of an actual medical apparatus. In particular, all or a predetermined selection of the functions of the actual medical apparatus can be performed by operating the virtual keypad. The 3D model of the medical apparatus executes the instructed functions. Therefore, a remote E-learning is possible.

In another advantageous implementation of the method, it further comprises the steps: capturing an image of the medical apparatus by the camera, and determining the type of the medical apparatus by evaluating the captured image of the real medical apparatus.

By this implementation, a manual determination of the type of the medical apparatus is not necessary since, from the captured image of the real medical apparatus, its type can be unambiguously determined. Moreover, the medical apparatus in its actual configuration can be determined and further information resulting thereof can be displayed.

According to a further advantageous implementation of the method, the information comprises a 3D model of a subassembly of the medical apparatus or of a component of the medical apparatus, and the method further comprises the step: rotating the 3D model of the subassembly or of the component by virtually touching the 3D model of the subassembly or of the component by means of an object and by moving the object.

By this characteristic, in case of maintenance or of a service call, components of the medical apparatus can easily be identified by visually investigating them from different views without lavishly opening the physical medical apparatus.

According to a further advantageous implementation of the method, the information comprises at least one of a service manual and a user manual of the medical apparatus, and the method further comprises the step: selecting an area in the service manual or the user manual by virtually touching the area by means of an object, and, thereby, providing further data about this area.

When selecting a specific area in the service manual, the further data can be, e.g., a video for assembling or dismantling a subassembly or instructions concerning, e.g., screw torques. The further data when selecting areas in the user manual can for example be videos of certain functions of the medical apparatus or instructions for attaching or detaching further components of the medical apparatus. Due to these data, maintenance and service calls or operation of the medical apparatus can be facilitated and the learning about the medical apparatus is improved.

**In** another advantageous implementation of the method, the computer assisted reality system comprises a transmission and reception device for transmitting data to and receiving data from the real medical apparatus, and the method comprises the step: exchanging data between the smart device and the medical apparatus by the transmission and reception device.

By this transmission and reception device, current data can be transmitted from the medical apparatus to the smart device, and data and instructions can be transmitted from the smart device to the medical apparatus.

According to a further advantageous implementation of the method, the information comprises the virtual keypad of the medical apparatus, and the method further comprises the step: actuating motions of the real medical apparatus according to inputs to the virtual keypad via the transmission and reception device.

The virtual keypad of the medical apparatus enables an operation corresponding to the operation of an actual medical apparatus. In particular, all of the functions of the actual medical apparatus can be performed by operating the virtual keypad and the real table performs these functions. Therefore, in case of a maintenance or service call, the medical apparatus can easily be operated by the smart device.

In a further advantageous implementation of the method, the information comprises an identifier of the medical apparatus, the identifier comprises at least one of the serial number of the medical apparatus and a firmware release of the software, the identifier is either input to the smart device or detected by evaluating the data transmitted from the medical apparatus, and the smart device retrieves data according to the identifier from a memory of the smart device or from the medical apparatus.

By being informed about the serial number of the medical apparatus or the firmware release of the software by the smart device, an exact determination of the functions of the medical apparatus or a determination of components or subassemblies is easily possible by retrieving the according data.

In a further advantageous implementation of the method, a controller of the medical apparatus determines state variables, and the information comprises at least one of the state variables transmitted to the smart device.

These state variables can be, for example, a height or a displacement of a tabletop of a surgical table as the medical apparatus so that a current posture of the tabletop can be determined. Furthermore, the state variables can relate to a time-dependent predictive maintenance alert so that the proper handling of, e.g., the surgical table is enhanced.

According to a further advantageous implementation of the method, the medical apparatus comprises sensors, a controller of the medical apparatus collects detected values of sensors of the medical apparatus, and the information comprises the detected values of the sensors.

The detected values of the sensors can indicate, e.g., whether a current overload of the surgical table as the medical apparatus or of components of the surgical table happens. Furthermore, the sensor values can relate to internal states, such as temperatures of electric drives or mechanical loads to any component, and/or to time of operation so that, e.g., a history can be recorded and predictive maintenance alert can be initiated or the states can be evaluated for analysis and complaint documentation.

According to a further advantageous implementation of the method, the computer assisted reality system comprises a remote device, the smart device comprises a transmission and reception device for transmitting data to and receiving data from the remote device, and the method comprises the step: exchanging data with the remote device by the transmission and reception device.

By this transmission and reception device, current data can be transmitted from the remote device to the smart device, and instructions can be transmitted from the smart device to the remote device which can be, e.g., a data base or an automated service bot. Further, this characteristic enables remote service by a distant diagnostic.

In a further advantageous implementation of the method, the information comprises a fault message when at least one of the state variables and one of the detected value of the sensors of the medical apparatus are beyond an allowable range, and the method further comprises the step: transmitting the fault message to the remote device.

When the fault message is transmitted to the remote device, e.g., faults of the same type of medical apparatus can be evaluated by a service ticketing system which may be linked to a complaint management system implemented in the remote device and, as the case may be, the evaluation can assist in providing a complaint documentation and in evaluating root causes.

According to a further advantageous implementation of the method, it further comprises the step: receiving data of a response to the fault message from the remote device by the smart device and displaying the response via the illustration device.

By displaying the response of the remote system via the illustration device, more exact data can be provided since fault messages of a plurality of the medical apparatuses of same type may be considered, in particular, for the preventive maintenance alert or for complaint management.

In a further advantageous implementation of the method, the information comprises at least one of a 3D model of a subassembly of the medical apparatus and a bill of material of the subassembly, and the method further comprises the step: selecting a component of the subassembly by virtually touching the component in the bill of material of the subassembly by means of an object, and transmitting a request concerning the selected component to the remote device by the smart device.

By this function, components of the medical apparatus can be selected easily for, e.g., ordering a spare part or for gathering additional data about this component.

According to a further aspect of the invention, a computer assisted reality system comprises a medical apparatus and a smart device having a camera and an illustration device for illustrating an image, wherein the computer assisted reality system is configured to execute a method for operation of the computer assisted reality system for the medical apparatus.

By this computer assisted reality system, the basis for learning about and training of the medical apparatus and for enhanced maintenance or service calls, i.e., the opportunity for input of according instructions, is given so that the proper handling is improved.

In an advantageous implementation of the computer assisted reality system, the medical apparatus is formed by a surgical table.

The surgical table has plenty of functions which partially depend on each other so that training of the operation of the surgical table is an important measure for the proper handling during use and, also, due to the complexity of the structure of the surgical table, assistance in maintenance and service improves the proper handling in this situation.

According to another aspect of the invention, a computer program product is configured to perform a method for operating of the augmented reality system for the medical apparatus.

By this computer program product, the basis for learning about and training of the medical apparatus and for enhanced maintenance or service calls, i.e., the opportunity for input of according instructions, is given so that the proper handling is improved.

Subsequently, the invention is elucidated by means of embodiments referring to the attached drawings.

In particular,
- Fig. 1: shows a structural view of an augmented reality system as an embodiment of a computer assisted reality system according to the invention;
- Fig. 2: shows an image of a 3D model of a surgical table as a medical apparatus and of a virtual keypad on a touchscreen of a smart device;
- Fig. 3: shows smart glasses as a further embodiment of the computer assisted reality system; and
- Fig. 4: shows a flowchart of a method according to the invention.

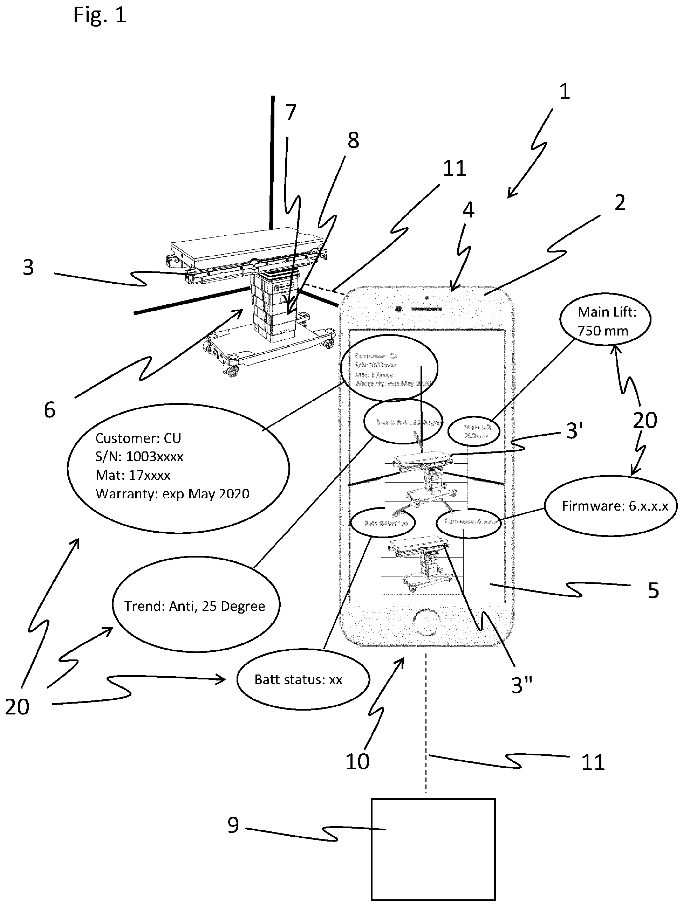

**Fig. 1** shows an augmented reality system 1 as an embodiment of a computer assisted reality system. The augmented reality system 1 comprises a smart device 2 and a surgical table 3 as the medical apparatus. In alternative embodiments, the medical apparatus is formed by, e.g., a surgical lamp, a surgical camera system or medical ceiling mounted support systems.

The smart device 2 comprises a camera 4 and a touchscreen 5 as an illustration device. Moreover, the smart device 2 is provided with a processor configured to execute a computer program performing a method for operation of the augmented reality system 1. This method enables an operator to use or investigate the surgical table 3 supported by images captured by the camera 4. At least one of the surgical table 3 and a backdrop is captured by the camera 4 and, therefore, a quasi-real situation is displayed on the touchscreen 5.

On the touchscreen 5, an image captured by the camera 4 is shown. In particular, an image of surroundings of the smart device 2 is captured by the camera 4 and displayed as a backdrop on the touchscreen 5. In this case, a corner 6 of a room is shown as the surroundings. Additionally or alternatively, an image of a real one of the surgical table 3' captured by the camera 4 is displayed and, moreover, an illustration of a 3D model of a type of the surgical table 3" is shown against the backdrop on the touchscreen 5. The image of the real one of the surgical table 3' can be displayed either against the real surroundings as the backdrop or, e.g., in a white frame as the backdrop.

Except from the images of the surgical table 3, information 20 concerning the surgical table 3 is displayed on the touchscreen 5. In the shown example, elements of information 20, such as an indication of a customer ("Customer"), an identifier, i.e., in this case, a serial number ("S/N"), a material number ("Mat") identifying the type of the surgical table 2, and an expiration date of the warranty ("Warranty"), are displayed. A further information 20 is the indication of the release of the firmware ("Firmware").

Furthermore, the touchscreen 5 is configured for input of instructions or data by touching the touch screen 5 by an object, e.g., a finger.

The surgical table 3 comprises a controller 7 and sensors 8 detecting various states of the surgical table 3. In the shown embodiment, at least a sensor 8 detecting a battery status is provided. The detected value of the sensor ("Batt status") is displayed on the touchscreen 5.

On the touchscreen 5, various state variables determined by the controller 7, such as a variable indicating the tilting ("Trend"), in the shown embodiment, 25° in the Anti-Trendelenburg direction, and an extension of a column of the surgical table 3 ("Main Lift"), as 750 mm, are displayed.

In alternative embodiments, not all of these elements of information 20 are displayed and/or other elements of information 20 are displayed on the touchscreen 5.

Moreover, a remote device 9 is provided and the smart device 2 comprises a transmission and reception device 10. The transmission and reception device 10 provides a respective transmission path 11 for transmitting data to and receiving data from, on the one hand, the surgical table 3 and, on the other hand, the remote device 9. In alternative embodiments, the transmission path 11 is only either provided for data transmission between the smart device 2 and the surgical table 3 or between the smart device 2 and the remote device 9.

In use, the augmented reality system 1 provides the opportunity to virtually operate or investigate the surgical table 3 as the medical apparatus based on an image captured by the camera 4 of the smart device 2.

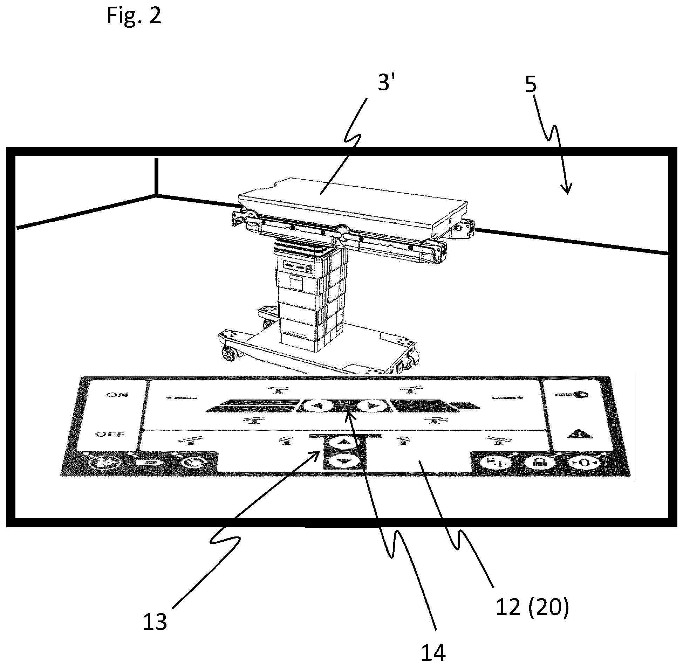

**Fig. 2** shows an image of the 3D model of the surgical table 3' as the medical apparatus and of a virtual keypad 12 on the touchscreen 5.

The virtual keypad 12 comprises keys for actuating motions of the shown image of the 3D model of the surgical table 3'. The motions are actuated according to inputs to the virtual keypad 12, i.e., according to the touched virtual keys. In this embodiment, keys 13 for a height adjustment of the tabletop of the surgical table 3 are provided. Furthermore, further keys 14 for a horizontal displacement of the tabletop of the image of the 3D model of the surgical table 3' in its longitudinal direction are provided.

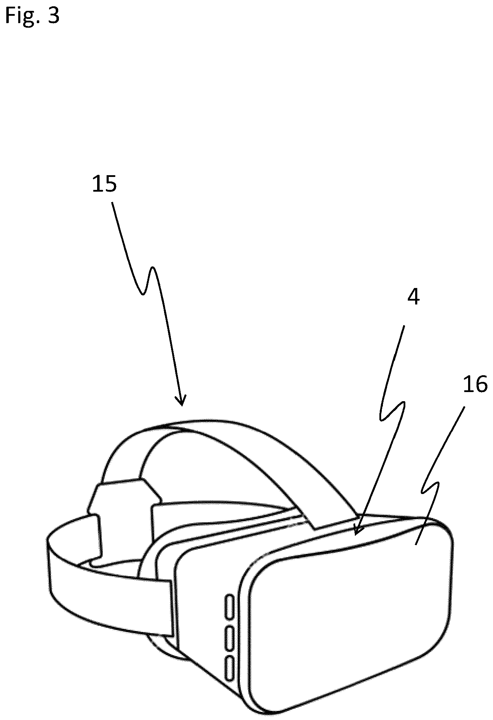

**Fig. 3** shows smart glasses 15 as a smart device of further embodiment of a computer assisted reality system in the form of a mixed reality system.

The smart glasses 15 comprise a screen 16 through which the surroundings can be observed by seeing through the screen 16. In the screen 16, a layer is provided as the illustration means for illustrating the 3D model of the surgical table 3' and the information in the manner of a head-up display. Further, the smart glasses 15 comprise the camera 4. In an alternative embodiment, another structure of the screen is possible.

The mixed reality system distinguishes from the augmented reality system in that it does not comprise the touch screen 5 by which images captured by the camera 4 are illustrated and by which input to the smart device is performed. Instead, inputs can be received by capturing the object and a motion of the object by the camera 4. The object, i.e., the finger or a hand, approaches the virtually illustrated 3D model of the type of the medical apparatus and either moves it by virtually touching the illustration of the 3D model or performs other activities, such as actuating a function via the virtual keypad.

The information corresponds to the information described with respect to the augmented reality system. Also, the transmission and reception device according 10 to that of the augmented reality system is provided.

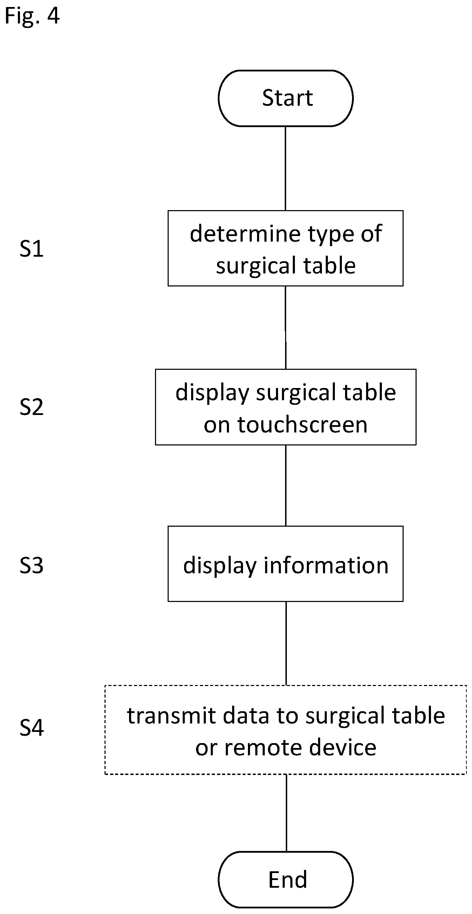

**Fig. 4** shows a flowchart of a method according to the invention.

In use of the augmented reality system 1 as the computer assisted reality system for a virtual operation of the surgical table 3 as the medical apparatus, e.g., for E-learning or for a service diagnostic, in a first step S1, the type of the surgical table 3 is determined. This determination happens by input of the type of the surgical table 3 by means of the touchscreen 5. Alternatively, the type of the surgical table 3 can also be determined by evaluating an image of the surgical table 3 captured by the camera 4 or by evaluating data received by the smart device 2 from the surgical table 3 via the transmission path 11.The evaluation of the image of the surgical table 3 takes place by comparing specific characteristics of the captured surgical table 3 with characteristics stored in a data base, e.g., in a memory of the smart device 2.

As the case may be, the image of the surroundings of the smart device 2 is captured as the backdrop and the image of the real surgical table 3 is captured. Alternatively, an image of merely one of the surroundings and the surgical table 3 is captured and the image of the real surgical table is illustrated against virtual surroundings or a virtual surgical table is illustrated against an image of the real surroundings.

In a second step S2, an image of the surgical table 3 according to the type of the surgical table 3 is displayed on the touchscreen 5. In particular, either the image of the real one of the surgical table 3' captured by the camera 4 is displayed or the image of the 3D model of the type of the surgical table 3" or both of the images are displayed against the backdrop. Alternatively, the real backdrop is not displayed but the image of the 3D model is shown in a white frame.

The image of the 3D model of the type of the surgical table 3" can be moved on the touchscreen 5 by touching the image of the 3D model of the type of the surgical table 3" on the touchscreen 5 by means of the object, e.g., the finger, and by moving the object on the touchscreen 5. Depending on the available degrees of freedom, the image of the 3D model of the surgical table 3" can be displaced and/or rotated on the touchscreen 5. Moreover, optionally, the image of the 3D model of the surgical table 3" can be up-scaled or downscaled.

Furthermore, in step S3, the information 20 concerning the surgical table 3 is displayed on the touchscreen 5.

The displayed information 20 comprise the virtual keypad 12, a 3D model of at least one of a subassembly of the surgical table 3 and a component of the surgical table 3, a bill of material of a subassembly of the surgical table 3, the service manual of the surgical table 3, the user manual of the surgical table 3, at least one of the identifiers of the surgical table 3, at least one of the state variables of the surgical table 3, at least one of the detected value of the sensors 8, and a fault message. In alternative embodiments, not all of these elements of the information 20 are displayed or further elements of the information 20 are displayed on the touchscreen 5.

Optionally, the data of the smart device 2 is transmitted to or exchanged with the surgical table 3 or transmitted to or exchanged with the remote device 9 by the transmission and reception device 10. The remote device 9 is a network, such as the Internet, wherein various services are offered in the network. Alternatively, the remote device 9 may be a single computer or a local network.

The virtual keypad 12 is used for actuating motions of the 3D model of the surgical table 3" according to inputs to the virtual keypad 12. By touching corresponding symbols of the virtual keypad 12, the motions of the 3D model of the surgical table 3" are actuated. Optionally, the real surgical table 3 can be controlled such that motions of the real surgical table 3 are actuated according to the inputs to the virtual keypad 12.

The displayed 3D model of the at least one of a subassembly of the surgical table 3 and a component of the surgical table 3 can be rotated on the touchscreen 5 by touching this 3D model on the touchscreen 5 by means of the object and by moving the object.

When selecting a component of the subassembly of the surgical table 3 by touching the touchscreen 5 by means of the object, a request concerning the selected component is transmitted to the remote device 9. This may be an order for a spare part of this component. The component can be selected by touching either the 3D model including the component or a line in the bill of material.

In the displayed service manual or user manual of the surgical table 3, an area thereof is selected by touching by means of the object. By selecting this area, further data about this area can be provided. This is, e.g., a video for assembling or disassembling a selected subassembly or further data or instructions concerning this subassembly. Alternatively, other data about this area is provided.

From the displayed identifier which, in the shown embodiment, includes the serial number of the surgical table 3 and a software release of the software of the surgical table 3, the surgical table can unambiguously be identified by the smart device 2. Alternatively, only one or another identifier is indicated. This identifier is either input to the touchscreen, e.g., by means of a virtual numerical or alphanumerical key panel, or it is detected by evaluating the data transmitted from the surgical table. Then, the smart device 2 retrieves data according to the identifier from a memory of the smart device 2, from the surgical table 3, or from the remote device 9.

Displaying of the state variables enables, for example, an exact determination of a posture of the tabletop of the surgical table 3. Moreover, the state variables may be based on evaluated working conditions and a date of a next preventive maintenance can be indicated thereof.

Displaying of detected value of sensors transmitted to the smart device 2 enables a detection of current working conditions, such as a temperature of an electric motor or a battery status.

If a fault occurs, i.e., if at least one of a critical state variable or a detected value of a critical sensor is beyond an allowable range, the fault message is displayed on the touchscreen 5. Such a fault message can also be signaled by means of an acoustic signal. This fault message is also transmitted to the remote device 9. In an alternative embodiment, the fault message is only displayed by the touchscreen 5.

The smart device 2 may receive data of a response to the fault message sent to the remote device 9 from the remote device 9. This response is displayed on the touchscreen 5. Such a response is generated, e.g., by an automated service bot or help desk, when an appropriate countermeasure for, e.g., remedying a fault is available. Moreover, if a problem cannot be resolved by the service bot, based on the state variable or value of a critical sensor transmitted to the remote device 9, a service case can be escalated and human service operators can deal with the problem online.

In case of such a fault or any technical problem, a group chat communication between technicians is possible by transmitting and sharing files, firmware, images, or videos via the smart device 2.

While the invention has been illustrated and described in detail in the drawings and the foregoing description, such illustration and description are to be considered illustrative or exemplary and not restrictive. The invention is not limited to the disclosed embodiments. From reading the present disclosure, other modifications will be apparent to a person skilled in the art. Such modifications may involve other features, which are already known in the art and may be used instead of or in addition to features already described herein. In the claims, the word "comprising" does not exclude other elements or steps, and the indefinite article "a" or "an" does not exclude a plurality.

Embodiments of the disclosure can be described with reference to the following numbered clauses, with additional features laid out in the dependent clauses:
Clause 1. A method of operation for a computer assisted reality system (1) for a medical apparatus, wherein the computer assisted reality system (1) comprises the medical apparatus and a smart device (2) having a camera (4) and an illustration device for illustrating an image, the method comprising the steps:
   determining a type of the medical apparatus by detecting an input of the type to the smart device or by evaluating an image of a real medical apparatus captured by the camera (4); and
   displaying information (20) concerning the medical apparatus via the illustration device.
Clause 2. The method of clause 1, wherein the computer assisted reality system is formed by an augmented reality system and the illustration device is formed by a touchscreen (5) illustrating an image captured by the camera (4), wherein
   the input to the smart device is performed via touching the touchscreen (5) by an object, comprising the step:
   illustrating an illustration of the medical apparatus according to the type of the medical apparatus against a backdrop via the illustration device.
Clause 3. The method of clause 2, further comprising the step:
   capturing an image of surroundings of the smart device (2) by the camera (4); and
   illustrating an illustration of a 3D model of the type of the medical apparatus in the image of the surroundings being the backdrop via the illustration device.
Clause 4. The method of clause 3, further comprising the steps:
   capturing an image of the medical apparatus by the camera (4);
   illustrating the captured image of the real medical apparatus against the backdrop; and
   determining the type of the medical apparatus by evaluating the captured image of the real medical apparatus.
Clause 5. The method of clause 1, wherein
   the computer assisted reality system is formed by a mixed reality system and the illustration device is formed by smart glasses (15),
   the input to the smart device is performed via capturing an object and a motion of the object by the camera (4), and the method comprises the step:
      illustrating an illustration of a 3D model of the type of the medical apparatus via the illustration device.
Clause 6. The method of anyone of clauses 3 to 5, further comprising the step:
   moving the illustration of the 3D model of the type of the medical apparatus by virtually touching the illustration of the 3D model of the type of the medical apparatus via the illustration device by means of the object and by moving the object.
Clause 7. The method of anyone of clauses 3 to 6, wherein
   the information (20) comprises a virtual keypad (12) of the medical apparatus, and
   the method further comprises the step:
      actuating motions of the 3D model of the medical apparatus according to inputs to the virtual keypad (12).
Clause 8. The method of any preceding clause, further comprising the steps:
   capturing an image of the medical apparatus by the camera (4); and
   determining the type of the medical apparatus by evaluating the captured image of the real medical apparatus.
Clause 9. The method of any preceding clause, wherein
   the information (20) comprises a 3D model of a subassembly of the medical apparatus or of a component of the medical apparatus, and
   the method further comprises the step:
      rotating the 3D model of the subassembly or of the component illustrated by the illustration device by virtually touching the 3D model of the subassembly or of the component via the illustration device by means of an or the object and by moving the object.
Clause 10. The method of any preceding clause, wherein
   the information (20) comprises at least one of a service manual and a user manual of the medical apparatus, and
   the method further comprises the step:
      selecting an area in the service manual or the user manual by virtually touching the area by means of an or the object, and, thereby, providing further data about this area.
Clause 11. The method of any preceding clause, wherein the computer assisted reality system (1) comprises a transmission and reception device (10) for transmitting data to and receiving data from the real medical apparatus, and
   the method further comprises the step:
   exchanging data the between the smart device (2) and the medical apparatus by the transmission and reception device (10).
Clause 12. The method of clause 11, wherein
   the information (20) comprises a or the virtual keypad (12) of the medical apparatus, and
   the method further comprises the step:
      actuating motions of the real medical apparatus according to inputs to the virtual keypad (12) via the transmission and reception device (10).
Clause 13. The method of clause 11 or 12, wherein
   the information (20) comprises an identifier of the medical apparatus,
   the identifier comprises at least one of a serial number of the medical apparatus and a firmware release of the software,
   the identifier is either input to the smart device or detected by evaluating data transmitted from the medical apparatus, and
   the method further comprises the step:
      retrieving data according to the identifier from a memory of the smart device (2) or from the medical apparatus by the smart device (2) by means of the transmission and reception device (10).
Clause 14. The method of anyone of clauses 11 to 13, wherein
   a controller (7) of the medical apparatus determines state variables, and
   the information (20) comprises at least one of the state variables transmitted to the smart device (2) by the transmission and reception device (10).
Clause 15. The method of anyone of clauses 11 to 14, wherein
   the medical apparatus comprises sensors (8) ,
   a or the controller (7) of the medical apparatus collects detected values of the sensors (8), and
   the information (20) comprises the detected values of the sensors (8) transmitted to the smart device (2) by the transmission and reception device (10).
Clause 16. The method of any preceding clause, wherein
   the computer assisted reality system (1) comprises a remote device (9),
   the smart device (2) comprises a transmission and reception device (10) for transmitting data to and receiving data from the remote device (9), and the method comprises the step:
      exchanging data with the remote device (9) by the transmission and reception device (10) .
Clause 17. The method of clause 16, wherein
   the information (20) comprises a fault message when at least one of one of state variables and one of detected values of the sensors (8) of the medical apparatus are beyond an allowable range, and
   the method further comprises the step:
      transmitting the fault message to the remote device (9).
Clause 18. The method of clause 17, further comprising the step:
   receiving data of a response to the fault message from the remote device (9) by the smart device (2); and
   displaying the response via the illustration device.
Clause 19. The method of anyone of clauses 16 to 18, wherein
   the information (20) comprises at least one of a 3D model of a subassembly of the medical apparatus and a bill of material of the subassembly, and
   the method further comprises the step:
      selecting a component of the subassembly by virtually touching the component in the bill of material of the subassembly by means of an object, and
      transmitting a request concerning the selected component to the remote device (9) by the smart device (2) .
Clause 20. A computer assisted reality system (1) comprising a medical apparatus and a smart device (2) having a camera (4) and an illustration device for illustrating an image, wherein the computer assisted reality system (1) is configured to execute a method according to anyone of the preceding clauses.
Clause 21. The computer assisted reality system of clause 20, wherein
   the medical apparatus is formed by a surgical table (3).
Clause 22. A computer program product configured to perform a method according to anyone of the clauses 1 to 19.

## Claims

1. A method of operation for a computer assisted reality system (1) for a medical apparatus, wherein the computer assisted reality system (1) comprises the medical apparatus and a smart device (2) having an illustration device for illustrating an image, the method comprising the steps:
determining a type of the medical apparatus by detecting an input of the type to the smart device; and
displaying information (20) concerning the medical apparatus via the illustration device, wherein the information comprises a 3D model of the type of the medical apparatus.

2. The method of claim 1, wherein the medical apparatus is a surgical table.

3. The method of claim 1 or 2, wherein the information further comprises a virtual keypad, optionally wherein the method further comprises at least one of:
actuating motions of the 3D model of the medical apparatus according to inputs to the virtual keypad (12); and
controlling motions of the medical apparatus according to inputs to the virtual keypad (12).

4. The method of claim 3 when dependent on claim 2, wherein the keypad includes keys for:
a height adjustment of a tabletop of the surgical table (3); and
a horizontal displacement of the tabletop of the image of the 3D model of the surgical table (3') in its longitudinal direction.

5. The method of any preceding claim, wherein the smart device comprises a physical button and/or wherein the 3D model displays an actual configuration of the medical apparatus.

6. The method of any preceding claim, wherein the smart device comprises a camera, and wherein the method comprises capturing an image of surroundings of the smart device (2) by the camera (4), and illustrating an illustration of the 3D model of the type of the medical apparatus in the image of the surroundings being the backdrop via the illustration device.

7. The method of any preceding claim, wherein the illustration device is formed by a touchscreen.

8. The method of claim 7, wherein the information (20) further comprises a 3D model of a subassembly of the medical apparatus or of a component of the medical apparatus, and the method further comprises the step: rotating the 3D model of the subassembly or of the component illustrated by the illustration device by virtually touching the 3D model of the subassembly or of the component via the illustration device by means of an or the object and by moving the object.

9. The method of claim 7 or 8, wherein determining a type of the medical apparatus by detecting an input of the type to the smart device is based on input of the type of medical apparatus by means of the touchscreen.

10. The method of any preceding claim, wherein the information (20) further comprises at least one of a service manual and a user manual of the medical apparatus, and the method further comprises the step: selecting an area in the service manual or the user manual by virtually touching the area by means of an or the object, and, thereby, providing further data about this area.

11. The method of any preceding claim, wherein the computer assisted reality system (1) comprises a transmission and reception device (10) for transmitting data to and receiving data from the real medical apparatus, and the method further comprises the step: exchanging data the between the smart device (2) and the medical apparatus by the transmission and reception device (10), and
optionally wherein the medical apparatus comprises sensors (8), a controller (7) of the medical apparatus collects detected values of the sensors (8), and the information (20) comprises the detected values of the sensors (8) transmitted to the smart device (2) by the transmission and reception device (10).

12. The method of claim 11, wherein the input of the type to the smart device detected for determining the type of the medical apparatus is data received by the smart device from the medical apparatus, and determining a type of the medical apparatus by detecting an input of the type to the smart device comprises evaluating the data received by the smart device from the medical apparatus.

13. The method of claim 11 or 12, wherein the information (20) comprises an identifier of the medical apparatus, the identifier comprising at least one of a serial number of the medical apparatus and a firmware release of software of the medical apparatus, wherein: the identifier is either input to the smart device or detected by evaluating data transmitted from the medical apparatus, and the method further comprises the step: retrieving data according to the identifier from a memory of the smart device (2) or from the medical apparatus by the smart device (2) by means of the transmission and reception device (10).

14. A computer assisted reality system (1) comprising a medical apparatus and a smart device (2) having an illustration device for illustrating an image, wherein the computer assisted reality system (1) is configured to execute a method according to any one of the preceding claims, optionally wherein the medical apparatus is formed by a surgical table (3).

15. A computer program product configured to perform a method according to any one of the claims 1 to 13.
